# EUROPÄISCHE PATENTSCHRIFT

(11) **EP 1 476 105 B1**
(45) Veröffentlichungstag und Bekanntmachung des Hinweises auf die Patenterteilung: **27.07.2005**
(21) Anmeldenummer: 03742526.1
(22) Anmeldetag: 17.02.2003
(51) Int. Cl.: A61F 13/00

(54) **SCHEIBEN-BZW. PAD-FÖRMIGER FASERVERBUNDARTIKEL**
DISK OR PAD-SHAPED FIBRE COMPOSITE ARTICLE
ARTICLE RENFORCE PAR DES FIBRES SE PRESENTANT SOUS FORME DE DISQUE OU DE PLAQUETTE

(30) Priorität: 20.02.2002 DE 10207721
(43) Veröffentlichungstag der Anmeldung: 17.11.2004
(73) Patentinhaber: Paul Hartmann Aktiengesellschaft, 89522 Heidenheim (DE)
(72) Erfinder: MANGOLD, Rainer, 89542 Herbrechtingen (DE); RÖMPP, Angela, 73119 Zell u.A. (DE); MICHELMANN, Jana, 89522 Heidenheim (DE)
(74) Vertreter: Friz, Oliver
(86) Internationale Anmeldenummer: PCT/EP2003/001551
(87) Internationale Veröffentlichungsnummer: WO 2003/070134

(56) Entgegenhaltungen:
- EP-A- 1 136 024
- WO-A-01/51548
- US-A- 5 910 225
- US-B1- 6 214 362

## Beschreibung

Die vorliegende Erfindung betrifft einen scheiben- bzw. padförmigen Faserverbundartikel für die Pflege und Reinigung der menschlichen Haut, mit einer eine Oberfläche des Artikels bildenden Faservlieslage und mit einer ebenfalls eine Oberfläche bildenden thermoplastischen Folienlage.

Derartige Artikel werden üblicherweise als Wattepad bezeichnet. Ein Wattepad der vorstehend beschriebenen Art ist beispielsweise aus US-B1-6214 362 und aus DE 200 05 170 U1 bekannt. Die thermoplastische Beschichtung bei diesem lotztgenannten bekannten Wattepad ist mit einer abrasiven Oberfläche versehen, indem in diese thermoplastische Folienlage ein Feststoffgranulat eingebunden ist. Hierdurch sollen Hautunreinheiten oder Hautschuppen leichter entfernt werden können, und zwar auch unter Verwendung einer schleifmittelfreien Reinigungsmilch oder Peeling-Creme.

WO 00/33974 zeigt ein Wattepad mit einer Faservlieslage, einer daran angrenzenden Sperrschicht, die aus einem thermoplastischen Film oder einem mit thermoplastischem Material beschichteten Papier gebildet sein kann. An diese Sperrschicht angrenzend ist eine Oberflächenschicht mit einer als Handgriff dienenden Faltung vorgesehen, die nach einem Ausführungsbeispiel der Druckschrift aus "ADVANTECH 2000 Synthetic Paper" gebildet sein kann, bei dem es sich wohl um ein Polypropylen umfassendes Papier handelt. Selbst wenn man diese oberflächenbildende Schicht als thermoplastische Folienlage bezeichnen wollte, so stellt sie keine Gebrauchsschicht dar, sondern dient lediglich zum Halten des Wattepads.

Ein ähnliches Wattepad ist aus EP 0 527 779 B1 bekannt. Sowohl die Grifflasche als auch die Sperrschicht können aus einem geeigneten thermoplastische Folienmaterial gebildet sein. Nach einer Ausführungsform kann die Haltelasche in ihre Gebrauchsform geklappt werden, so dass ein Oberflächenbereich der darunter angeordneten Sperrschicht freigegeben wird, die in der Verpackungslage der Grifflasche verdeckt ist. Wiederum bildet aber nur die gegenüberliegende Faservlieslage, diejenige Seite, die zum bestimmungsgemäßen Reinigen der Haut Verwendung finden soll. Es ist in dieser Druckschrift darauf hingewiesen, dass die Sperrschicht flüssigkeits- und partikelundurchlässig sein soll.

Ein ähnliches Wattepad zeigt DE 199 16 640 A1.

Es ist ferner ein auf dem Markt erhältliches Kosmetikprodukt bekannt, welches aus einer einzigen thermoplastischen fettabsorbierenden Folienlage besteht. Dieses Produkt kann für die Hautpflege und -reinigung Verwendung finden.

Ausgehend vom eingangs genannten Stand der Technik liegt der vorliegenden Erfindung die Aufgabe zugrunde, einen Faserverbundartikel in Form eines sogenannten Wattepads dahingehend zu verbessern, dass er bei der Pflege und Reinigung der menschlichen Haut in vielfältiger Weise einsetzbar ist. Er soll insbesondere verschiedenen, in der Praxis auftretenden Anforderungen gerecht werden, die nicht nur vom jeweiligen Benutzer sondern auch von verschiedenen Einsatzzwecken, wie z. B. Abreinigen von kosmetischen Substanzen, Schmutz oder fettigen Bestandteilen der Haut, abhängen.

Diese Aufgabe wird bei einem Wattepad der genannten Art erfindungsgemäß dadurch gelöst, dass die thermoplastische Folienlage zur Aufnahme von fettigen Bestandteilen der menschlichen Haut eine poröse Struktur aufweist.

Mit der Erfindung wird also vorgeschlagen, eine thermoplastische poröse Folienlage in einem Wattepad als Oberflächenschicht zum Wischen bzw. Reinigen der menschlichen Haut, nicht jedoch nur als Flüssigkeitsbarriere oder Anfasshilfe zu verwenden. Auf diese Weise wird das Wattepad vielfältiger einsetzbar. So ist es also möglich, die oberflächenexponierte Faservlieslage in an sich bekannter Weise zum Auftragen oder Entfernen von kosmetischen Substanzen, aber auch zur Reinigung der Hautoberfläche zu benutzen. Die ebenfalls oberflächenexponierte thermoplastische Folienlage kann indessen bevorzugt zur Entfernung von fettigen Bestandteilen der menschlichen Haut eingesetzt werden. Diese fettigen Bestandteile werden dann von der eher hydrophoben und damit lipohilen Folienlage und deren Poren aufgenommen. Beispielsweise kann zunächst die Faservlieslage zum Einsatz kommen, und daran anschließend kann der Artikel beispielsweise gefaltet werden, indem die gebrauchte Faservliesoberfläche auf sich selbst gefaltet wird, so dass auf der anderen Seite die thermoplastische Folienlage zum anschließenden Reinigen der Haut von Fettbestandteilen zum Einsatz kommen kann. Vorzugsweise kommt zuerst die Folienlage zum Entfernen von Fettbestandteilen zum Einsatz. Anschließend werden mit der Faservliesoberseite Schminkkorrekturen vorgenommen, z.B. Nachpudern oder dergleichen.

Es hat sich als vorteilhaft erwiesen, wenn das thermoplastische Polymer ein Polyolefin, insbesondere Polyethylen (PE) oder Polypropylen (PP) oder eine Mischung hieraus ist. Ferner erweist es sich als vorteilhaft, wenn die thermoplastische Folienlage einen vorzugsweise anorganischen Füllstoff enthält. Zur Herstellung einer zur Aufnahme von fettigen Bestandteilen geeigneten porösen thermoplastischen Folienlage wird ein thermoplastisches Polymer mit einem vorzugsweise anorganischen Füllstoff gemischt, wobei aus dem Gemisch in an sich bekannter Weise eine Folie hergestellt wird, die dann gestreckt wird, wodurch die Poren entstehen.

Alternativ wäre es aber auch denkbar, dass die füllstoffhaltige Folie durch Kalandrieren mit Poren versehen wird. Wenn vorstehend von einer porösen Folienlage gesprochen wird, so wird hierunter eine solche Folienlage verstanden, die oftmals auch als Mikroporen bezeichnete Öffnungen einer Porengröße von weniger als 50 µm umfasst. Hierbei soll nicht ausgeschlossen werden, dass in der Folienlage auch größere Poren ausgebildet sein können. Indessen erweist es sich als vorteilhaft, wenn die Poren überwiegend eine Größe von weniger als 50 µm, insbesondere von 0,5 bis 50 µm, oder 0,5 bis 40 µm und bevorzugtermaßen von 0,5 bis 30 µm oder 0,5 - 20 µm, insbesondere 0,5 - 10 µm umfassen.

Derartige Folien sind in Form des eingangs genannten, auf dem Markt erhältlichen Produkts auch bekannt. Ebenso können thermoplastische Folienlagen Verwendung finden, wie sie beispielsweise in EP 0 293 482 B1 oder in DE 33 06 843 A1 und in US-A-4,585,604 als Backsheetmaterialien für absorbierende Hygieneartikel beschrieben sind.

Es wäre denkbar und könnte sich auch als vorteilhaft erweisen, wenn die Faservlieslage und die Folienlage auf derselben Seite des Artikels vorgesehen wären und so jeweils einen Teil oder Bereich der zur Reinigung der Haut bestimmten Seite des Artikels bilden würden. Die betreffenden Oberflächenbereiche könnten dann insbesondere flächenbündig nebeneinander angeordnet sein. Es wird aber auch eine Ausführungsform bevorzugt, bei der die Faservlieslage auf der der Folienlage gegenüberliegenden Seite des Artikels angeordnet ist, so wie dies vorausgehend schon angedeutet wurde.

Für die Ausbildung der Faservlieslage bestehen an sich keine besonderen Restriktionen; es erweist sich als vorteilhaft, wenn ein als sanft und weich empfundenes Faservliesmaterial verwendet wird. Hierfür kommt bevorzugtermaßen Baumwolle in Frage, welches Material in mehrfacher Hinsicht als vorteilhaft und geeignet anzusehen ist. Die Faservlieslage kann aber auch thermoplastische Fasern umfassen und unter Anschmelzen der thermoplastischen Fasern thermisch verfestigt sein. Eine besondere Bedeutung kommt nämlich der Integrität der Faservlieslage in einem Wattepad der hier interessierenden Art zu. So stellt die thermische Verfestigung von Wattepads neben an sich auch denkbaren Kompaktierungsverfahren, wie Kalandrieren, oder Wasserstrahlvernadeln, ein denkbares und vorteilhaftes Mittel zur Vliesverfestigung dar.

Als besonders bevorzugt hat sich eine Zusammensetzung der Faservlieslage erwiesen, wonach diese zu 5 bis 20 Gew.-% thermoplastische Fasern und gegebenenfalls insbesondere bis zu 95 Gew.-% Baumwollfasern umfasst.

Als besonders bevorzugt hat sich ferner eine Zusammensetzung der Faservlieslage erwiesen, wonach diese synthetische Mikrofasern, z.B. Viskose- oder Polyesterfasern, einer Faserstärke von unterhalb 1 dtex umfasst, insbesondere in Mischung mit Baumwollfasern. Mikrofasern sind insbesondere zu 40 bis 90 Gew.-% in der Faservlieslage enthalten.

Aber auch eine Faservlieslage, die zu 100% aus Baumwollfasern besteht, ist denkbar und vorteilhaft. Insbesondere in diesem Fall eignet sich bevorzugtermaßen der Einsatz der Wasserstrahlverfestigung von einer oder von beiden Seiten der Faservlieslage, um das Faservlies in sich zu verfestigen, so dass es ohne zu zerfasern manuell gegriffen und benutzt werden kann.

Der Bereich vorteilhafter Flächengewichte der Faservlieslage liegt bei 50 bis 260 g/m². Unter einem Prüfdruck von 0,5 kPa hat sich eine Vliesdicke von 1,2 mm bis 5 mm als vorteilhaft erwiesen.

Gegenstand der vorliegenden Erfindung ist mithin auch die Verwendung einer an sich bekannten porösen thermoplastischen Folienlage als Oberflächenschicht in einem eine Faservliesschicht aufweisenden Faserverbundartikel zur Aufnahme von fettigen Bestandteilen der menschlichen Haut bei der Hautreinigung oder -pflege.

Die Flächengewichte der thermoplastischen Folienlage betragen zwischen 20 und 80 g/m², und deren Dicke liegt zwischen 30 und 70 µm.

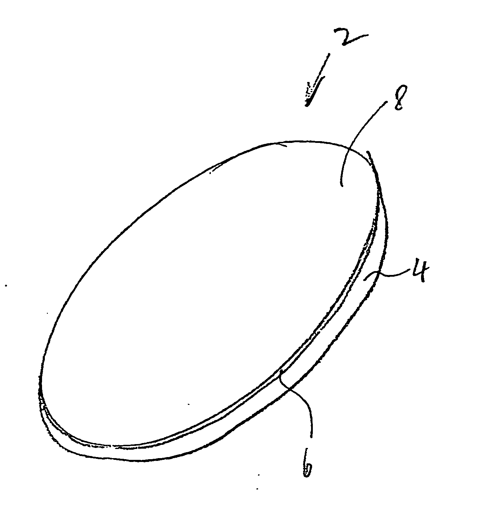

Weitere Merkmale, Einzelheiten und Vorteile der Erfindung ergeben sich aus den beigefügten Patentansprüchen sowie der zeichnerischen Darstellung und nachfolgenden Beschreibung einer bevorzugten Ausführungsform des erfindungsgemäßen Faserverbundartikels. In der Zeichnung zeigt:

Die Figur eine perspektivische Ansicht eines insgesamt mit dem Bezugszeichen 2 bezeichneten Wattepads mit einer Faservlieslage 4 aus beispielsweise 100% Gew.-% Baumwolle oder aus beispielsweise 85 Gew.-% Baumwolle und 15 Gew.-% thermoplastischen Bikomponentenfasern. Im letzteren Fall sind die Fasern homogen gemischt und die so erhaltene Faservlieslage ist durch Anschmelzen der gegenüber einer Kernkomponente niedriger schmelzenden Mantelkomponente der Bikomponentenfasern thermisch verfestigt. Bei einer im wesentlichen ausschließlich aus Baumwollfasern bestehenden Faservlieslage erweist es sich als vorteilhaft, wenn diese beispielsweise durch Wasserstrahlvernadeln in sich verfestigt ist. Bei beiden Ausführungsformen kann die Faservlieslage auf ihrer in der Figur nicht ersichtlichen Gebrauchsseite ein Rillenmuster oder Waffelmuster oder dergleichen strukturierte Oberflächengestaltung aufweisen.

An die der Gebrauchsseite gegenüberliegende Seite der Faservlieslage 4 ist eine Folienlage 6 aus einem thermoplastische Polymer, vorzugsweise auf Polyolefinbasis, z.B. PE oder PP, mit einem anorganischen Füllstoff angefügt.

Die Folienlage kann beispielsweise mittels eines Heißschmelzklebers oder durch andere dem Fachmann zur Verfügung stehende Mittel an die Faservlieslage angefügt sein.

Bei der Folienlage handelt es sich um mikroporöse Folien mit Poren im Bereich von vorzugsweise 0,5 bis 40 µm, insbesondere 0,5 bis 30 µm und vorzugsweise 0,5 - 20 µm oder 0,5 - 10 µm Porengröße. Ihr Flächengewicht beträgt 20 bis 80 g/m² und ihre Dicke liegt zwischen 30 und 70 µm. Im dargestellten Fall bildet die der Faservlieslage 4 abgewandte Oberseite 8 der Folienlage eine weitere Gebrauchsseite, die direkt mit der Hautoberfläche in Kontakt bringbar ist. Beim Abwischen der Hautoberfläche werden bevorzugt fettige Bestandteile der menschlichen Haut in der porösen Struktur der Folienlage 6 aufgenommen. Der so erhaltene Wattepad lässt sich daher zur Reinigung der Haut von fettigen Bestandteilen benutzen, wobei die herkömmlichen Anwendungszwecke bekannter Wattepads über die Gebrauchsseite der Faservlieslage 4 nach wie vor und zusätzlich zur Verfügung stehen.

## Patentansprüche

1. Scheiben- bzw. pad-förmiger Faserverbundartikel (2) für die Pflege und Reinigung der menschlichen Haut, mit einer eine Oberfläche des Artikels bildenden Faservlieslage (4) und mit einer ebenfalls eine Oberfläche bildenden thermoplastischen Folienlage (6), **dadurch gekennzeichnet, dass** die Faservlieslage (4) unter Zusatz thermoplastischer Fasern thermisch verfestigt ist und/oder synthetische Mikrofasern einer Faserstärke von weniger als 1 dtex umfasst, und dass die thermoplastische Folienlage (6) zur Aufnahme von fettigen Bestandteilen der menschlichen Haut eine poröse Struktur aufweist.

2. Faserverbundartikel nach Anspruch 1, **dadurch gekennzeichnet, dass** das thermoplastische Polymer ein Polyolefin, insbesondere Polyethylen (PE) oder Polypropylen (PP), ist.

3. Faserverbundartikel nach Anspruch 1 oder 2, **dadurch gekennzeichnet, dass** die thermoplastische Folienlage (6) einen Füllstoff aufweist.

4. Faserverbundartikel nach Anspruch 3, **dadurch gekennzeichnet, dass** der Füllstoff anorganisch ist.

5. Faserverbundartikel nach einem oder mehreren der vorstehenden Ansprüche, **dadurch gekennzeichnet, dass** die thermoplastische Folienlage (6) Poren einer Größe von 0,5 - 50 µm, insbesondere von 0,5 - 40 µm, insbesondere von 0,5 - 30 µm, insbesondere von 0, 5 - 20 µm und insbesondere von 0,5 - 10 µm umfasst.

6. Faserverbundartikel nach einem oder mehreren der vorstehenden Ansprüche, **dadurch gekennzeichnet, dass** die Faservlieslage (4) auf der der Folienlage (6) gegenüberliegenden Seite des Artikels (2) angeordnet ist.

7. Faserverbundartikel nach einem oder mehreren der vorstehenden Ansprüche, **dadurch gekennzeichnet, dass** die Faservlieslage (4) von einem Wattevlies auf Basis von Baumwollfasern gebildet ist.

8. Faserverbundartikel nach einem oder mehreren der vorstehenden Ansprüche, **dadurch gekennzeichnet, dass** die thermoplastischen Fasern Bikomponentenfasern sind.

9. Faserverbundartikel nach einem oder mehreren der vorstehenden Ansprüche, **dadurch gekennzeichnet, dass** die Faservlieslage (4) zu 5 - 20 Gew.-% thermoplastische Fasern umfasst.

10. Faserverbundartikel nach einem oder mehreren der vorstehenden Ansprüche **dadurch gekennzeichnet, dass** die Faservlieslage (4) zu 40 - 90 Gew.-% Mikrofasern umfasst.

11. Faserverbundartikel nach einem oder mehreren der vorstehenden Ansprüche, **dadurch gekennzeichnet, dass** die Faservlieslage (4) bis zu 95 Gew.-% Baumwollfasern umfasst.

12. Faserverbundartikel nach einem oder mehreren der vorstehenden Ansprüche, **dadurch gekennzeichnet, dass** die Faservlieslage (4) wasserstrahlverfestigt ist.

## Claims

1. Disc-or-pad shaped composite fibre article (2) for the care and cleaning of human skin, comprising a non-woven fibre layer (4) forming a surface of the article and comprising a thermoplastic film layer (6) also forming a surface, **characterised in that** the non-woven fibre layer (4) is thermally solidified with the addition of thermoplastic fibres and/or comprises synthetic microfibres that have a thickness of less than 1 dtex, and **in that** the thermoplastic film layer (6) has a porous structure for absorbing greasy constituents of the human skin.

2. Composite fibre article according to claim 1, **characterised in that** the thermoplastic polymer is a polyolefin, in particular polyethylene (PE) or polypropylene (PP).

3. Composite fibre article according to either claim 1 or claim 2, **characterised in that** the thermoplastic film layer (6) comprises a filler.

4. Composite fibre article according to claim 3, **characterised in that** the filler is inorganic.

5. Composite fibre article according to one or more of the preceding claims, **characterised in that** the thermoplastic film layers (6) comprises pores having a size of 0.5 to 50 µm, in particular of 0.5 to 40 µm, in particular of 0.5 to 30 µm, in particular of 0.5 to 20 µm and in particular of 0.5 to 10 µm.

6. Composite fibre article according to one or more of the preceding claims, **characterised in that** the non-woven fibre layer (4) is arranged on that side of the article (2) which is opposite the film layer (6).

7. Composite fibre article according to one or more of the preceding claims, **characterised in that** the non-woven fibre layer (4) is formed by non-woven wadding based on cotton fibres.

8. Composite fibre article according to one or more of the preceding claims, **characterised in that** the thermoplastic fibres are bi-component fibres.

9. Composite fibre article according to one or more of the preceding claims, **characterised in that** the non-woven fibre layer (4) comprises 5 to 20 % by weight of thermoplastic fibres.

10. Composite fibre article according to one or more of the preceding claims, **characterised in that** non-woven fibre layer (4) comprises 40 to 90 % by weight of microfibres.

11. Composite fibre article according to one or more of the preceding claims, **characterised in that** non-woven fibre layer (4) comprises up to 95 % by weight of cotton fibres.

12. Composite fibre article according to one or more of the preceding claims, **characterised in that** non-woven fibre layer (4) is solidified by means of a water jet.

## Revendications

1. Article composite fibreux (2) en forme de disque ou de tampon pour le soin et le nettoyage de la peau humaine, avec une couche de non tissé (4) formant une surface de l'article et une couche de feuille thermoplastique (6) formant également une surface, **caractérisé en ce que** la couche de non tissé (4) est consolidée thermiquement en ajoutant des fibres thermoplastiques et/ou comprend des microfibres synthétiques d'une épaisseur de fibres de moins de 1 dtex et **en ce que** la couche de feuille thermoplastique (6) présente une structure poreuse pour absorber des composants gras de la peau humaine.

2. Article composite fibreux selon la revendication 1, **caractérisé en ce que** le polymère thermoplastique est une polyoléfine, en particulier du polyéthylène (PE) ou du polypropylène (PP).

3. Article composite fibreux selon la revendication 1 ou 2, **caractérisé en ce que** la couche de feuille thermoplastique (6) comporte un matériau de charge.

4. Article composite fibreux selon la revendication 3, **caractérisé en ce que** le matériau de charge est minéral.

5. Article composite fibreux selon l'une ou plusieurs des revendications précédentes, **caractérisé en ce que** la couche de feuille thermoplastique (6) comporte des pores d'une grosseur comprise entre 0,5 et 50 µm, en particulier entre 0,5 et 40 µm, en particulier entre 0,5 et 30 µm, en particulier entre 0,5 et 20 µm et en particulier entre 0,5 et 10 µm.

6. Article composite fibreux selon l'une ou plusieurs des revendications précédentes, **caractérisé en ce que** la couche de non tissé (4) est agencée sur la face de l'article (2) opposée à la couche de feuille (6).

7. Article composite fibreux selon l'une ou plusieurs des revendications précédentes, **caractérisé en ce que** la couche de non tissé (4) est formée par un non tissé d'ouate à base de fibres de coton.

8. Article composite fibreux selon l'une ou plusieurs des revendications précédentes, **caractérisé en ce que** les fibres thermoplastiques sont des fibres à deux composants.

9. Article composite fibreux selon l'une ou plusieurs des revendications précédentes, **caractérisé en ce que** la couche de non tissé (4) comprend de 5 à 20 % en poids de fibres thermoplastiques.

10. Article composite fibreux selon l'une ou plusieurs des revendications précédentes, **caractérisé en ce que** la couche de non tissé (4) comprend de 40 à 90 % en poids de microfibres.

11. Article composite fibreux selon l'une ou plusieurs des revendications précédentes, **caractérisé en ce que** la couche de non tissé (4) comprend jusqu'à 95 % en poids de fibres de coton.

12. Article composite fibreux selon l'une ou plusieurs des revendications précédentes, **caractérisé en ce que** la couche de non tissé (4) est consolidée par jet d'eau.
